**Europäisches Patentamt**

**European Patent Office**

**Office européen des brevets**

(19)

(11) Veröffentlichungsnummer: **0 118 927**
**B1**

(12) # EUROPÄISCHE PATENTSCHRIFT

(45) Veröffentlichungstag der Patentschrift:
18.03.87

(51) Int. Cl.⁴: **A 61 B 10/00,** A 61 B 17/34

(21) Anmeldenummer: 84104804.4

(22) Anmeldetag: 28.04.84

(54) **Biopsiekanüle.**

(30) Priorität: 12.11.83 DE 3341117

(43) Veröffentlichungstag der Anmeldung:
19.09.84 Patentblatt 84/38

(45) Bekanntmachung des Hinweises auf die Patenterteilung:
18.03.87 Patentblatt 87/12

(84) Benannte Vertragsstaaten:
AT BE CH FR GB IT LI NL SE

(56) Entgegenhaltungen:
DE-A-1 957 661
GB-A-2 042 902
GB-A-2 099 703
US-A-4 403 617

(73) Patentinhaber: **Schnepp- Pesch, Wolfram,
Schönblick 6, D-7505 Ettlingen (DE)**
Patentinhaber: **Lindenberg, Josef, Buchenweg 13,
D-7512 Rheinstetten 4 (DE)**

(72) Erfinder: **Schnepp- Pesch, Wolfram, Schönblick 6,
D-7505 Ettlingen (DE)**
Erfinder: **Lindenberg, Josef, Buchenweg 13, D-7512
Rheinstetten 4 (DE)**

(74) Vertreter: **Dr.- Ing. Hans Lichti Dipl.- Ing. Heiner
Lichti Dipl.- Phys. Dr. Jost Lempert, Postfach 41
07 60 Durlacher Strasse 31, D-7500 Karlsruhe 41
(DE)**

**Beschreibung**

Die Erfindung betrifft eine Biopsiekanüle mit einer einen axialen Hohlraum zur Aufnahme einer Gewebeprobe umgebenden Zylindermantel-Wand und mindestens einer Schneide mit zwei unter einem spitzen Winkel in einer Schneidkante endenden Schneidflanken.

Die Biopsie dient zur Entnahme von Gewebeproben, wie aus Nieren, Prostata, Brust, Leber,Lunge, zur histologischen Untersuchung. Zur Biopsie wird wenigstens eine Biopsienadel verwandt, die aus einer Biopsiekanüle und einem Stilett besteht. Bei einer bekannten Biopsienadel (Tru-Cut der Fa. Travenol AG) weist das Stilett eine sich im Bereich seines vorderen Endes erstreckende längere Kerbe auf. Die Spitze ist durch Abschrägung des Endes des Stiletts gebildet. Die Kanüle weist ebenfalls ein abgeschrägtes Ende mit einer abgerundeten Schneide am äußersten Ende auf. Die Biopsie wird derart vorgenommen, daß bei in die Kanüle weitgehend eingezogenem Stilett die Biopsienadel entweder bis vor oder bis in das zu entnehmende Gewebe eingestochen wird. Anschließend wird die Kerbe freigegeben, indem entweder die Kanüle zurückgezogen oder aber das Stilett weiter vorgeschoben wird. Die Probenkerbe wird hierdurch freigelegt, so daß in sie von der Seite her Gewebe eindringen kann. Das in die Kerbe eingedrungene Gewebe wird dadurch geschnitten, daß die Kanüle über die Kerbe geschoben wird.

Anschließend wird die gesamte Biopsienadel in geschlossener Position zurückgezogen. Mit dieser bekannten Biopsienadel kann insofern zuverlässig gearbeitet werden, als eine ausreichende Gewebemenge ohne größere Gefahr der Zerstörung der Zellen entnommen werden kann. Aufgrund der Schwächung des Stiletts durch die in diesem vorgesehene Kerbe muß aber der Durchmesser des Stiletts und damit der gesamten Nadel recht groß gewählt werden und liegt im Bereich von nahezu 2mm. Hierdurch werden in den zu biopsierenden Organen oder Gewebebereichen erhebliche Läsionen bei der Biopsie verursacht, die zu Hämatomen führen. Eine Biopsie mit dieser bekannten Biopsienadel ist für den Patienten mit erheblichen Schmerzen verbunden, was dazu führt, daß Patienten ansich notwendige Biopsien zu vermeiden trachten.

Bei einem weiteren Biopsiebesteck weist die Biopsienadel ein Stilett mit angespitztem Ende und eine gattungsgemäße Biopsiekanüle auf, die in ihrem Endbereich entsprechend der Form der Spitze des Stiletts auf ihrem äußeren Umfang mit drei schrägen Schleifflächen versehen ist (Franseen Lungenbiopsienadel). Diese Spitzenform der Kanüle dient zum besseren Einführen zusammen mit dem Stilett, da hierdurch praktisch vom Stilett zur Kanüle eine einheitliche Einführspitze in Form einer dreieckigen Pyramide gebildet wird. Diese Chiba-Biopsienadel dient zur sogenannten Menghini-Saugbiopsie. Hierbei wird nach Einführen der Nadel das Stilett aus der Kanüle herausgenommen. Anschließend wird die Kanule an ihrem äußeren Ende mit einer Saugspritze versehen, die aufgezogen wird, wodurch Gewebebröckelchen in die Nadel hereingesaugt werden. Die Nadel kann zwar aufgrund der Einsatzweise recht dünn gewählt werden und einen Durchmesser im Bereich von weniger als 1 mm aufweisen, beim Einziehen der Gewebsbröckelchen werden aber lediglich mehr oder minder zufällig ganze Zellen mit eingezogen. Demgegenüber ist die Gefahr erheblich, daß Zellen zerstört werden können, was insbesondere bei bösartigen Zellen bekanntermaßen mit herheblichen Risiken verbunden ist.

Eine Kanüle nach der DE-A-19 57 661 nach Jamshidi zeigt zunächst ein längeres konisches distales Ende, durch welches beim Einführen Gewebe zur Seite gedrückt wird. Das distale Ende ist abgeschrägt oder mit in axialer Richtung spiralförmig ansteigenden Schneidkanten jeweils um den halben Umfang versehen. Die US-A-4 403 617 zeigt ein Biopsiebesteck mit einseitig abgeschrägter Nadel und eine Kanüle mit ebenfalls konisch zulaufendem Endbereich, dessen stirnseitige Schneidkante wellenförmig ausgebildet und mit einem Außenschliff versehen ist, so daß die äußere Schneidflanke von der Außenwandfläche der Kanüle in einem spitzen Winkel auf die Innenwandfläche zuläuft und mit dieser als innere Schneidflanke die Schneidkante bildet.

Der Erfindung liegt die Aufgabe zugrunde, eine Biopsiekanüle für Biopsienadeln zu schaffen, die unter Vermeidung der genannten Nachteile des dargelegten Standes der Technik mit minimalstem Durchmesser ausgebildet werden kann, gleichzeitig aber die Entnahme einer ausreichenden sauber geschnittenen Gewebeprobe ohne die Gefahr der Zerstörung von Zellen ermöglicht.

Erfindungsgemäß wird die genannte Aufgabe durch eine Biopsiekanüle der eingangs genannten Art gelöst, welche derart ausgebildet ist daß die erste, äußere Schneidflanke Teil der äußeren Fläche der Zylindermantel-Wand ist, während die zweite, innere Schneidflanke eine geschliffene Fläche ist, die sich von der inneren Fläche der Zylindermantel-Wand derart schräg nach außen fortsetzt, bis sie die äußere Fläche der Zylndermantel-Wand und damit die äußere Schneidflanke in der sich im wesentlichen achsparallel erstreckenden Schneidkante unter einem spitzen Winkel schneidet, und daß die Zylindermantel-Wand eine durch die innere Schneidflanke gebildete und von dieser einseitig begrenzte Ausnehmung aufweist.

Kern der Erfindung ist also, daß nicht, wie es bisher regelmäßig der Fall war, neben Nadeln auch die Kanülen auf ihrem äußeren Umfang lediglich in eine gewünschte Form geschliffen wurden, sondern daß demgegenüber die Biopsiekanule an ihrem vorderen wirksamen Ende mit einem inneren Schliff versehen wird, daß also nicht die äußere Fläche des Zylindermantels der Kanüle geschliffen wird, sondern der Innenumfang oder die innere Fläche der Kanüle ausgeschliffen wird und hierdurch eine Schneidkante gebildet wird. Dabei kann auch zur Erzeugung einer gewünschten Schneidkante der äußere Umfang leicht angeschliffen sein, wesentlich ist aber daß durch das Ausschleifen der Innenwand eine Schneidkante weit zum äußeren Umfang hin verlegt wird. In bevorzugter Ausgestaltung ist vorgesehen, daß die von innen nach außen verlaufende Schneidflanke tangential von der inneren Fläche der Zylindermantel-Wand fort verläuft. Hierdurch wird im Bereich des Außenumfangs eine optimale Schneide durch einerseits der von innen nach außen geschliffenen Schneidflanke, andererseits der zweiten durch den Außenumfang gebildeten Schneidflanke und die Schneidkante, in der die beiden Schneidflanken zusammenlaufen. Die Schneidkante begrenzt dabei seitlich eine Ausnehmung, deren gegenüberliegende Begrenzungsfläche der Zylindermantel-Wand parallel zu der ausgeschliffenen Schneidflanke verlaufen kann, was eine äußerst einfache Herstellung in einem Arbeitsschritt ermöglicht. Die der Schneidflanke gegenüberliegende, die Ausnehmung begrenzende Wandfläche kann aber noch zusätzlich in gewünschter Weise geschliffen sein, beispielsweise in einer Form, die ein gewisses Eindringen des zu biopsierenden Gewebes seitlich in die der Schneidkante benachbarten Ausnehmung leichter erfolgen läßt. Während gemäß einen Merkmal der Erfindung die Schneidkante im wesentlichen achsparallel verläuft, ist sie in äußerst bevorzugter Ausgestaltung derart leicht geneigt, daß praktisch eine Hinterschneidung entsteht und der Winkel zwischen dem vorderen Umfangsrand der Kanüle und der Schneidkante wenige als 90° beträgt, vorzugsweise im Bereich von etwa 85° liegt.

Zur Verwendung mit der erfindungsgemäßen Biopsiekanüle kann ein ansich herkömmliches Stilett, beispielsweise ein solches, wie es bei der Chiba-Biopsienadel eingesetzt wird, verwendet werden, wenn auch in dem Falle, daß sie an ihrer vorderen Stirnseite eine ringförmige in einer Ebene senkrecht zur Achse verlaufende Kante aufweist und insbesondere daß sie an ihrem vorderen Ende in der Form des Mantels eines Kegelstumpfes ausgebildet ist, ein Stilett mit einer kegelstumpfförmig zugeschliffenen Spitze zu bevorzugen ist.

Mit der erfindungsgemäßen Biopsiekanüle wird folgendermaßen gearbeitet: Die Biopsienadel aus der erfindungsgemäßen Biopsiekanüle und einem entsprechenden Stilett wird bei vollständig eingeschobenem Stilett durch die Haut des Patienten in den zu biopsierenden Bereich eingestochen.

Anschließend wird das Stilett aus der Biopsiekanüle herausgezogen und am äußeren Ende der Biopsiekanüle eine Saugspritze angesetzt. Diese wird geringfügig aufgezogen, so daß in der Biopsiekanüle ein leichter Unterdruck entsteht. Wichtig ist, daß sie nicht so weit aufgezogen wird, daß allein durch den Unterdruck schon Gewebebrocken in die Kanüle eingesogen werden, wie dies bei der Menghini-Saugbiopsie vorgesehen ist. Zu diesem Zweck ist es vorteilhaft, daß die Saugspritze in an sich bekannter Weise Markierungen oder Arretierungen aufweist, so daß je in Abhängigkeit vom Durchmesser der Biopsiekanüle der Kolben der Saugspritze nur um einen vorbestimmtan Weg herausgezogen und damit nur ein geringer vorbestimmter Unterdruck erzeugt wird. Anschließend wird die erfindungsgemäße Biopsiekanüle bei angelegtem geringem Unterdruck unter Drehung leicht nach vorne geschoben und durch die Drehung wird von der erfindungsgemäß ausgebildeten Schneidkante Gewebe fortlaufend spiralförmig aus dem zu biopsierendan Organ herausgeschnitten und aufgrund des Unterdruckes wird das geschnittene Gewebe in die Kanüle eingezogen. Anschließend wird die Kanüle herausgezogan.

Weitere Vorteile und Merkmale der Erfindung ergeben sich aus den Ansprüchen und aus der nachfolgenden Beschreibung, in der ein Ausführungsbeispiel der erfindungsgemäßen Biopsiekanüle im einzelnen erläutert ist. Dabei zeigt:

Figur 1 ein Ende der erfindungsgemäßen Biopsiekanüle in Seitenansicht; und

Figur 2 einen Schnitt entlang der Linie II - II der Kanüle nach der Figur 1.

Die dargesllte Ausführungsform einer erfindungsgemäßen Biopsiekanüle 1 weist einen Körperteil mit einer Zylindermantel-Wand 2 auf, die einen axialen Hohlraum 3 umgibt. Die Kanüle 1 ist an ihrem vorderen Ende 4 in der Form eines Kegelstumpfes 6 konisch verjüngt. Von der Stirnseite 7 der Kanüle 1 aus erstrecken sich im wesentlichen in axialer Richtung, mit einer ganz geringen Neigung zur Achse der Kanüle 1 zwei Schneiden 8, die diametral in der Zylindermantel-Wand 2 der Kanüle 1 ausgebildet sind. Die Schneiden 8 werden durch zwei spitz aufeinander zulaufende und in einer Schneidkante 9 endende Schneidflanken 11, 12 gebildet. Die Schneidflanke 12 ist dabei Teil der äußeren Fläche 13 der Zylindermantel-Wand 2, während die innere Schneidflanke 11 eine geschliffene Fläche ist, die sich tangential von der inneren Fläche 14 der Zylindermantel-Wand 2 nach außen fortsetzt, bis sie die äußere Fläche der Zylindermantel-Wand 13 und damit die äußere Schneidflanke 12 in der Schneidkante 9 schneidet. Derart wird auf dem äußeren Umfang der Zylindermantel-Wand 2 der Biopsiekanüle 1 die Schneide 12 gebildet. Die Schneidkante 9 verläuft, wie gesagt, nicht genau axial, sondern schließt zu einer axialen Mantellinie einen Winkel von wenigen Grad, bis zu 5° ein, indem die Schneidkante von der Stirnseite 7 gegenüber einer achsparallelen Linie auf den Mantel 2 zurückgeneigt ist. Insofern wird durch die ringförmige Stirnkante 16 der Schneidkanüle 1 und die Schneidkante 8 nicht ein Winkel von 90°, sondern von etwas unter 90° eingeschlossen. Hierdurch wird das Einschneiden der Schneidkante im Bereich der Stirnseite 7 der Kanüle 1 verbessert.

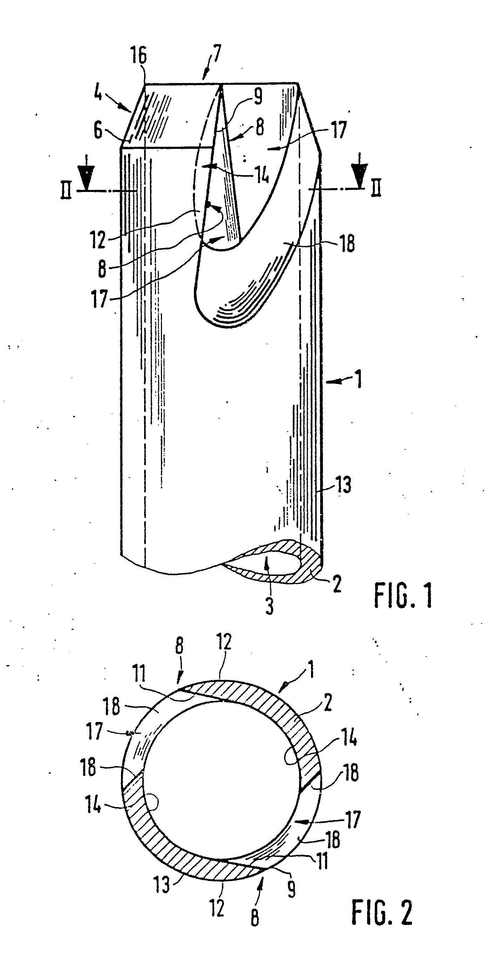

Die Schneide 8 begrenzt jeweils an einer axialen Seite eine Ausnehmung 17 in der Zylindermantel-Wand 2 der Schneidkanüle 1, wobei die Ausnehmung 17 an der Stirnseite 7 der Kanüle 1 offen ist und im übrigen durch Außen- und Innenfläche verbindende ausgeschliffene Wandflächen 18 begrenzt wird. Die Wandflächen 18 sind dabei, wie insbesondere in Figur 1 zu entnehmen ist, bogenförmig geschliffen. Die Schneide 8, damit die Schneidkante 9 und die Schneidflanke 11 können gemeinsam mit der Ausnehmung 17 und den anderen diesen begranzenden Wandteilen 18 geschliffen werden. Zusätzlich können aber weitere einzelne Bearbeitungs- bzw. Schleifschritte einerseits an der Schleifkante 11 und andererseits an der dieser gegenüberliegendan Wandfläche 18 vorganommen sein.

Die erfindungsgemäße Biopsiekanüle, wie sie in den Figuren 1 und 2 beschrieben ist, wird als Teil eines Biopsiebestecks folgendermaßen eingesetzt:

Die erfindungsgemäße Biopsiekanüle 1 bildet zusammen mit einem in sie einsteckbaren Stilett eine Biopsianadel. Das Stilett ragt dabei an dem in den Figuren 1 und 2 dargestelltan vorderen Ende der Biopsiekanüle 1 etwas aus dieser heraus. Die beschriebene Biopsienadeleinheit wird vom Arzt nach geeigneter Anästhesie durch die Haut eines Patienten so weit eingeschlossen, bis das vordere Ende 7 der Biopsiekanüle 1 in das zu biopsierende Gewebe gelangt, wobei der Einstechvorgang mit Hilfe von Ultraschall beobachtet und damit ultraschallgezielt durchgeführt werden kann. Anschließend wird das Stilett aus der Biopsiekanüle herausgenommen und die Biopsiekanüle an ihrem äußeren (nicht dargestelltan) Ende mit einem Vakuumsauggerät, im einfachsten Fall dem Kolben-Zylinderteil einer Spritze verbunden. Diese wird dann zur Erzeugung eines Unterdrucks in der Biopsiekanüle geringfügig aufgezogen, wobei sie Anzeigeeinrichtungen und Arretiermechanismen aufweisen kann, mit der, ggfls. in Abhängigkeit von der Nadelstärke die Höhe des Aufziehens des Kolbens und damit die Größe des erzeugten Unterdrucks angezeigt bzw. begrenzt wird. Anschliessend wird bei der in den Zeichnungen dargestellten Ausführungsform die Biopsiekanüle unter leichtem Druck im Uhrzeigersinn gedreht. Hierdurch wird durch die Schneide 8 ein dünner zylindrischer Gewebestreifen aus dem zu biopsierenden Gewebe herausgeschnitten und einerseits durch den leichten Vorschub, andererseits durch den Unterdruck in der Biopsiekanüle 1 in diese hineingezogen. Auch dieser Vorgang kann unter Ultraschall beobachtet und damit mit höchster Präzision durchgeführt werden. Nachdem ein ausreichend langer Streifen Gewebe ausgeschnitten und in die Biopsiekanüle 1 hineingezogen wurde, wird diese einfach aus dem Körper des Patienten wieder herausgezogen. Das herausgeschnittene, zu untersuchende Gewebe wird dann langsam aus der Biopsiekanüle herausgestoßen, indem der Kolben der Spritze od.dgl. vorsichtig wieder in dem Zylinderteil eingedrückt wird, wodurch das Gewebeteil aus dem Ende 7 der Biopsiekanüle 1 herausgedrückt wird. Es kann dann den üblichen histologischen Untersuchungen zugeführt werden. Die erfindungsgemäße Biopsiekanüle erlaubt es, einen äußerst dünnen Streifen von Gewebe aus dem zu biopsierenden Bereich herauszunehmen, ohne daß einerseits Zellen mit den hierdurch möglicherweise verbundenen nachteiligen Folgen zerstört werden und ohne daß andererseits größere Läsionen, die zu Hämatoman od.dgl. führen könnten, im biopsierten Gewebe zurückbleiben. Mittels der erfindungsgemäßen Biopsiekanüle kann eine Biopsie durchgeführt werden, die den Patienten wesentlich geringere Schmerzen als im Falle eines Herausschneidens von in die Ausnehmung eines Obturators hereingezogenen Gewebe aussetzt, dannoch aber nicht, wie bei der Menghini-Saugbiopsie die Gefahr der Zerstörung von Zellen bedingt.

**Patentansprüche**

1. Biopsiekanüle mit einer einen axialen Hohlraum (3) zur Aufnahme einer Gewebeprobe umgebenden Zylindermantel-Wand (2) und mindestens einer Schneide (8) mit zwei unter einem spitzen Winkel in einer Schneidkante (9) endenden Schneidflanken (11, 12), dadurch gekennzeichnet, daß die erste, äußere Schneidflanke (12) Teil der äußeren Fläche (13) der Zylindermantel-Wand (2) ist, während die zweite, innere Schneidflanke (11) eine geschliffene Fläche ist, die sich von der inneren Fläche (14) der Zylindermantel-Wand (2) derart schräg nach außen fortsetzt, bis sie die äußere Fläche (13) der Zylindermantel-Wand (2) und damit die äußere Schneidflanke (12) in der sich im wesentlichen achsparallel erstreckenden Schneidkante (9) unter einem spitzen Winkel schneidet, und daß die Zylindermantel-Wand (2) eine durch die innere Schneidflanke (11) gebildete und von dieser einseitig begrenzte Ausnehmung (17) aufweist.

2. Kanüle nach Anspruch 1, dadurch gekennzeichnet, daß die von innen nach außen verlaufende Schneidflanke (11) tangential von der inneren Fläche (14) der Zylindermantel-Wand (2) fort verläuft.

3. Kanüle nach Anspruch 1 oder 2, dadurch gekennzeichnet, daß die Schneidkante (9) leicht derart zu einer achsparallelen Mantellinie auf der Wand (2) geneigt ist, daß die Schneidkante (9) leicht übersteht und mit der Stirnseite der Zylindermantel-Wand (2) ein Winkel von weniger als 90° einschließt.

4. Kanüle nach Anspruch 3, dadurch gekennzeichnet, daß der Neigungswinkel der Schneidkante zu einer achsparallelen Mantellinie der Wand (2) etwa bei 5° liegt.

5. Kanüle nach einem der vorangehenden Ansprüche, dadurch gekennzeichnet, daß zwei Schneiden (8) diametral einander gegenüberliegend ausgebildet sind.

6. Kanüle nach einem der vorangehenden Ansprüche, dadurch gekennzeichnet, daß eine durch die Schneidkante (9) gebildete und von dieser einseitig begrenzte Ausnehmung im Mantel (2) eine Breite von etwa einem Viertel des Mantelumfangs (2) aufweist.

7. Kanüle nach einem der vorangehenden Ansprüche, dadurch gekennzeichnet, daß eine der Schneidkante (9) auf der anderen Seite der Ausnehmung (17) gegenüberliegende und diese begrenzende Wand (18) parallel zur inneren Schneidflanke (11) verläuft.

8. Kanüle nach einem der vorangehenden Ansprüche, dadurch gekennzeichnet, daß sie an ihrer vorderen Stirnseite eine ringförmige, in einer Ebene senkrecht zur Achse verlaufende Kante aufweist.

9. Kanüle nach Anspruch 8, dadurch gekennzeichnet, daß sie an ihrem vorderen Ende in der Form des Mantels eines Kegelstumpfes ausgebildet ist.

## Claims

1. Biopsy cannula with a cylinder jacket wall (2) surrounding an axial cavity (3) for receiving a tissue sample and at least one cutter (8) with two cutting flanks (11, 12) terminating at an acute angle in a cutting edge (9), characterized in that the first, outer cutting flank (12) is part of the outer surface (13) of the cylinder jacket wall (2), whilst the second, inner cutting flank (11) is a ground surface, which extends in outwardly sloping manner from the inner surface (14) of the cylinder jacket wall (2) until it intersects the outer surface (13) of the cylinder jacket wall (2) and therefore the outer cutting flank (12) in the substantially axially parallel cutting edge (9) under an acute angle and that the cylinder jacket wall (2) has a recess (17) formed by the inner cutting flank (11) and bounded by the same on one side.

2. Cannula according to claim 1, characterized in that the cutting flank (11) running from inside to outside extends tangentially from the inner surface (14) of cylinder jacket wall (2).

3. Cannula according to claims 1 or 2, characterized in that the cutting edge (9) inclines slightly to an axial parallel generatrix on wall (2) in such a way that the cutting edge (9) slightly projects and forms with the end face of the cylinder jacket wall (2) an angle of less than 90°.

4. Cannula according to claim 3, characterized in that the angle of inclination of the cutting edge is approximately 5° relative to an axially parallel generatrix of wall (2).

5. Cannula according to one of the preceding claims, characterized in that two cutters (8) are formed diametrically facing one another.

6. Cannula according to one of the preceding claims, characterized in that a recess formed by cutting edge (9) and bounded on one side by the latter has in jacket wall (2) a width of approximately one quarter of the jacket circumference.

7. Cannula according to one of the preceding claims, characterized in that a wall (18) facing cutting edge (9) on the other side of recess (17) and bounding the same is parallel to the inner cutting flank (11).

8. Cannula according to one of the preceding claims, characterized in that on its front end face it has an annular edge running in a plane at right angles to the axis.

9. Cannula according to claim 8, characterized in that it is constructed at its front end in the form of the jacket of a frustum.

## Revendications

1. Canule pour biopsie, avec une paroi cylindrique (2) entourant un espace vide axial (3) pour recevoir un échantillon de tissu, et au moins un tranchant (8) avec deux flancs de tranchant (11, 12) se terminant sous un angle aigu en formant une arête coupante (9), caractérisée en ce que le premier flanc de tranchant, extérieur, (12) fait partie de la surface extérieure (13) de la paroi cylindrique (2), tandis que le deuxième flanc de tranchant, intérieur, (11) est une surface polie qui s'étend obliquement de la surface intérieure (14) de la paroi cylindrique (2) vers l'extérieur, de telle façon et jusqu'à ce qu'elle coupe la surface extérieure (13) de cette paroi cylindrique (2) et, par conséquent, le flanc de tranchant extérieur (12) sous un angle aigu en formant l'arête coupante (9) qui s'étend dans une direction sensiblement parallèle à l'axe, et que la paroi cylindrique (2) comporte un évidement (17) formé par le flanc de tranchant intérieur (11) et limité d'un côté par ce flanc.

2. Canule selon la revendication 1, caractérisee en ce que le flanc de tranchant intérieur (11), qui s'étend de l'intérieur vers l'extérieur, s'étend tangentiellement de la surface intérieure (14) de la paroi cylindrique (2).

3. Canule selon la revendication 1 ou 2, caractérisée en ce que l'arête coupante (9) est légèrement inclinée sur la paroi cylindrique (2) par rapport à une génératrice parallèle à l'axe de telle façon que cette arête coupante fasse légèrement saillie et forme un angle de moins de 90° avec le côté frontal de la paroi cylindrique (2).

4. Canule selon la revendication 3, caractérisée en ce que l'angle d'inclinaison de l'arête coupante (9) par rapport à une génératrice de la paroi cylindrique (2) parallèle à l'axe est d'environ 5°.

5. Canule selon l'une des revendications précédentes, caractérisée en ce que deux tranchants (8) diamétralement opposés sont formés.

6. Canule selon l'une des revendications précédentes, caractérisée en ce qu'un évidement dans la paroi cylindrique (2) formé par l'arête coupante (9) et limité d'un côté par celle-ci a une largeur égale à environ un quart du périmètre de la paroi cylindrique (2).

7. Canule selon l'une des revendications précédentes, caractérisée en ce qu'une paroi (18) disposée de l'autre côté de l'évidement (17), en face de l'arête coupante (9), et limitant cet évidement, s'étend parallèlement au flanc intérieur (11) du tranchant (8).

8. Canule selon l'une des revendications précédentes, caractérisée en ce qu'elle comporte au niveau de son côté frontal antérieur une arête annulaire qui s'étend dans un plan perpendiculaire à l'axe.

9. Canule selon la revendication 8, caractérisée en ce qu'à son extrémité antérieure, elle a la forme d'un tronc de cône.

FIG. 1

FIG. 2